# EUROPEAN PATENT SPECIFICATION

(11) **EP 1 551 390 B1**
(45) Date of publication and mention of the grant of the patent: **02.04.2014**
(21) Application number: 03741701.1
(22) Date of filing: 21.07.2003
(51) Int. Cl.: A61K 31/365, A61K 31/429, A61K 9/00, A61K 47/22, A61P 33/10

(54) **Formulation comprising avermectins / milbemycins and levamisole in a pyrrolidone solvent**
Formulierung enthaltend Avermectine / Milbemycine und Levamisol in einem Pyrrolidon-Lösungsmittel
Formulation comprenant des avermectines / milbemycines et levamisole dans un solvant pyrrolidone

(30) Priority: 19.07.2002 NZ 52029502
(43) Date of publication of application: 13.07.2005
(60) Divisional application: 11187966.4
(73) Proprietor: Merial Limited, Harlow, Essex CM19 5QA (GB)
(72) Inventor: HOLMES, Robert William Lachlan, Birkenhead, 1310 Auckland (NZ); RAZZAK, Majid Hameed Abdul (Dr), Albany, 1311 Auckland (NZ); JINDAL, Kour Chand (Dr), Chennai, 600 020 Tamilnadu (IN); NILENDU, Sen, Chennai, 600 041 Tamilnadu (IN)
(74) Representative: Clyde-Watson, Zöe
(86) International application number: PCT/NZ2003/000157
(87) International publication number: WO 2004/009080

(56) References cited:
- WO-A-00/74489
- WO-A-01/51028
- WO-A-97/26895
- US-A- 6 054 140
- US-B1- 6 340 672
- REILLY PE, ISAACS JP: 'Adverse drug interactions of importance in veterinary practice', [Online] vol. 112, no. 2, 08 January 1983, VET. REC., pages 29 - 33 Retrieved from the Internet: <URL:http://www.ncbi.nlm.nih.gov/pubmed/682 9139> [retrieved on 2012-10-02]

## Description

### FIELD OF THE INVENTION

This invention relates to the field of veterinary pharmaceuticals and in particular to anthelmintic formulations including a combination of actives.

### BACKGROUND

Anthelmintics are an important tool for farmers seeking to improve the productivity of grazing cattle. The first class of modem broad-spectrum anthelmintic was the benzimidazoles introduced in the early 1960's, followed by levamisole and morantel in the late 1960's and finally the avermectins and milbemycins in the early 1980's.

| ***Anthelmintic*** | ***Year of Introduction*** | ***Main Active's in the Group*** |
|---|---|---|
| Benzimidazoles | Early 1960's | Thiabendazole, albendazole, fenbendazole, oxfendazole |
| Levamisole/Morantel | Late 1960's | Levamisole, morantel |
| Avermectins/Milbemycins | Early 1980's | Abamectin, ivermectin, moxidectin, doramectin, eprinomectin |

Parasite resistance has developed to each group of anthelmintic since they were introduced. Resistance to benzimidazole-based drenches is widespread throughout the world. Cases have been reported that involve resistance in all three major cattle parasites species: *Ostertagia, Trichostrongylus* and *Cooperia.*

Resistance to levamisole/morantel based drenches is well known but is less widespread than benzimidazole resistance.

In 1995, New Zealand researchers reported a strain of the worm parasite *Cooperia* that was resistant to both ivermectin (a member of the avermectin/milbemycin group) and to oxfendazole (a benzimidazole). In 1996, reports were published of an ivermectin resistant *Cooperia* strain that was cross-resistant to doramectin and moxidectin (also members of the avermectin/milbemycin group).

To prevent and manage the problem of anthelmintic resistance farmers have relied on various number of strategies including:
- minimizing anthelmintic use by only treating at strategically important times
- alternating the type of anthelmintic used
- using combinations of anthelmintics from different groups to reduce the potential of parasites to survive the treatment.

Orally administered combinations of benzimidazole and levamisole anthelmintics are well known, and have been used for many years.

However in recent years products based on actives selected solely from the avermectin/milbemycin groups have held the most significant share of the cattle anthelmintic market due to their high efficacy against the major production limiting parasite species, *Ostertagia.* The availability of easy to apply topical pour-on formulations has further extended their market dominance.

By contrast, levamisole-based products have been used on a much more limited basis. Despite their having good efficacy against *Cooperia,* the key dose limiting parasite of the avermectin/milbemycin group.

The table below shows that while each anthelmintic group has particular limitations against certain parasites, a combination of actives selected from the avermectin/milbemycin and levamisole groups would achieve two highly important goals:
- high efficacy against the key cattle parasites
- combination potency to help prevent parasites surviving the treatment

| *Anthelmintic Class* | *Cooperia Efficacy* | *Ostertagia Efficacy* |
|---|---|---|
| Levamisole | Good | Poor |
| Avermectin/Milbemycin | Poor | Good |
| Combination of both classes | Good | Good |

Despite this rationale for an easy to use product combining levamisole active with an avermectin/milbemycin active combinations have been difficult to formulate.

Previous attempts included the formulation of a double active formulation including levamisole and niclosamide. This was designed to target tapeworm and roundworm. This formulation however, was unsatisfactory as exposure to water made it too viscous to use.

Further it was found the differing pH requirements of levamisole and other anthelmintics made it difficult to formulate a stable product.

NZ 336139 and WO 00/74489 represents a recent attempt to formulate a combination avermectin/milbemycin and levamisole product.

To achieve co-existance within the formulation Nufarm relies on emulsion technology. The emulsion includes formulation including the levamisole in aqueous acidic phase and including an anthelmintic such as an avermectin or milbemycin in the lipophilic phase. A third active can be suspended in particulate form in the aqueous phase.

The disadvantage of this formulation is the need for the formulation to be shaken or agitated into an emulsion. In addition, the product is chemically complicated including 2 or 3 different phases.

The complicated nature of the formulation in NZ 336139 and WO 00/74489 is due in part to the different formulation requirements of the actives. Avermectins and milbemycins being substantially insoluble in water whereas levamisole is water soluble. In addition, levamisole has previously been found to require a pH of less than about 4 for stability while avermectins and milbemycin require a pH of about 6.6.

As this will be appreciated, in addition to the stability issues topical formulations have a tendency to cause skin irritation to the animal at the site of application. Accordingly, a formulation to be acceptable for topical use it must not cause excessive skin irritation.

Accordingly, there is a need for a stable, formulation capable of stably including avermectins or milbemycins together with levamisole.

In addition, it is desirable the formulation be suitable for topical use.

Compositions of avermectins or levamisole in a pyrrolidone solvent have for instance been disclosed in US 6, 340, 672.

### OBJECT

It is an object of the present invention to provide a stable anthelmintic formulation or one that will at least provide the public with a useful choice.

### STATEMENT OF INVENTION

In one aspect the invention relates to a stable formulation suitable for administration to animals including at least 2 actives wherein a first of the actives is selected from the group including the avermectins and the milbemycins and the second of said actives is levamisole, said actives being dissolved in a pyrrolidone solvent.

Preferrably the formulation may additionally include a co-solvent selected from the glycol ether group.

Preferably the avermectin or milbemycin is selected from the group including abamectin, doramectin, eprinomectin, ivermectin and moxidectin.

Preferably the pyrrolidone solvent is N-methyl pyrrolidone or 2-pyrrolidone.

More preferably the avermectin or milbemycin is present in the range of between 0.01 - 5% w/v.

Preferably levamisole is present in the range of between 1 - 30% w/v.

Preferably the formulation additionally includes at least one further medicament selected from the group comprising anthelmintics, dietary supplements, vitamins, mineral and other beneficial agents.

More preferably wherein the formulation additionally includes excipients including preservatives, stabilisers, flavorants, co solvents.

Preferably the formulation is suitable for topical, injectable or oral administration.

More preferably the formulation is suitable for topical administration.

More preferably the formulation does not cause unacceptable levels of skin irritancy when applied topically.

In a further related aspect the invention relates to a method of treating or preventing infection of cattle with *Cooperia* or *Ostertagia* by administering a formulation of the present invention.

The formulations of the present invention must be stable to be of commercial use. In this specification, a commercially acceptable anthelmintic formulation is one which is stable at room temperature for a period of at least 6 months. In conditions of accelerated testing, at 40°C, this requires the potency of the actives within the formulation to remain within specified and acceptable limits for 3 months.

Avermectins and milbemycins where used in this specification refer to a group of actives having anthelmintic activity. The avermectin group includes by way of example, avermectin, ivermectin, doramection and eprinomectin. The milbemycin group includes moxidectin.

Pyrrolidones solvents useable in this invention include, N-methyl-2-pyrrolidone, 2-pyrrolidone, 1-pyrrolidone, N-ethylene-2-pyrrolidone, 3, 3-dimethyl-2-pyrrolidone, N-ethyl-2-pyrrolidone, 5-dimethyl-2-pyrrolidone, N-ethoxy-2-pyrrolidone, and combinations thereof.

Levamisole is used in this specification includes levamisole base, levamisole phosphate together with other salts and forms.

The invention the subject of the present application is advantageous as it provides stable formulations including an avermectin or milbemycin in combination with levamisole. Further, the formulations retain each active in solution.

The formulations are monophosic and suitable to manufacture on a commercial scale. In addition, as both actives are in solution the formulations are physically stable. in that it does not separate out into separate phases either aqueous and lipophilic phases or liquid and solid phases. This enables the formulations the subject of this application to ne used without requiring agitation or shaking before use and greatly reduces the risk of differing concentrations of actives through the drum or other storage container.

In addition, as the formulation excludes water the issue of incompatible pH requirements is alleviated. Enabling the two actives to stability co-exist in a single phase.

### DESCRIPTION

A large number of studies were undertaken over a 4 year period to develop a stable anthelmintic formulation combining levamisole and avermectin/milbemycin. In these studies abamectin was used as the representative avermectin/milbemycin active, whilst levamisole, in its base form, was used as the representative levamisole/morantel active.

### Study 1

A number of potential formulations were prepared using a soya bean oil base and common excipients used in the preparation of topical anthelmintics.

| **Formulation 1** | | **Formulation 2** | |
|---|---|---|---|
| **Materials** | **%w/v** | **Materials** | **%w/v** |
| Abamectin | 1 | Abamectin | 1 |
| Levamisole | 20 | Levamisole | 20 |
| Benzyl alcohol | 5 | Benzyl alcohol | 5 |
| Capmul PG-8 | 20 | Capmul PG-8 | 20 |
| Isopropyl Palmitate | 10 | Isopropyl Myristate | 10 |
| Tween 80 | 2 | Tween 80 | 2 |
| Soya bean oil | q.v. | Soya bean oil | q.v. |

| **Formulation 3** | | **Formulation 4** | |
|---|---|---|---|
| **Materials** | **%w/w** | **Materials** | **%w/w** |
| Abamectin | 1 | Abamectin | 1 |
| Benzyl alcohol | 5 | Levamisole | 20 |
| Capmul PG-8 | 20 | Benzyl alcohol | 5 |
| Isopropyl Palmitate | 10 | Capmul PG-8 | 20 |
| Tween 80 | 2 | Isopropyl Myristate | 10 |
| Soya bean oil | q.v. | Soya bean oil | q.v. |

None of these formulations were stable when tested under conditions of elevated temperature. All formulations exhibited significant degradation of the abamectin component. Animal studies also demonstrated an unexpected degree of skin irritancy with hair loss at the point of application. These results indicated that an oil-base to the product may be unsuitable both from an irritancy and stability perspective.

### Study 2

A number of formulations were prepared using propylene glycol and glycol ethers, both common excipients used in veterinary drug formulation. These were then subjected to conditions of elevated temperature to determine their potential shelf stability. As a positive control for stability testing purposes a commercially available avermectin/milbemycin product, Ivomec® Plus Injection was used.

### Formulations

| | R20 | R27 | R28 | R29 | Ivomec® | Levipor® | Ivomec® Plus injection |
|---|---|---|---|---|---|---|---|
| Lev.base | 20.0 g | 20.0 g | 20.0 g | 20.0 g | -- | 20.0 g | -- |
| Abamectin | 1.0 g | 1.0 g | 1.0 g | 1.0 g | -- | -- | -- |
| Ivermectin | -- | -- | -- | -- | 0.5 g | -- | 3.0 g |
| Propylene Glycol | 50 g | 41 g | 50 g | 41 g | | | |
| Benzyl alcohol | -- | -- | 10 g | 10 g | | | |
| BHT | 0.2 g | 0.2 g | 0.2 g | 0.2 g | | | |
| IPA | -- | 4g | -- | 4g | | | |
| *DGMEE to | 100ml | 100ml | 100ml | 100ml | * No more details | | |

| | | | | | | | |
|---|---|---|---|---|---|---|---|
| *DGMEE: Diethylene glycol monoethyl ether (Transcutol ^{®}) | | | | | | | |

### Stability results

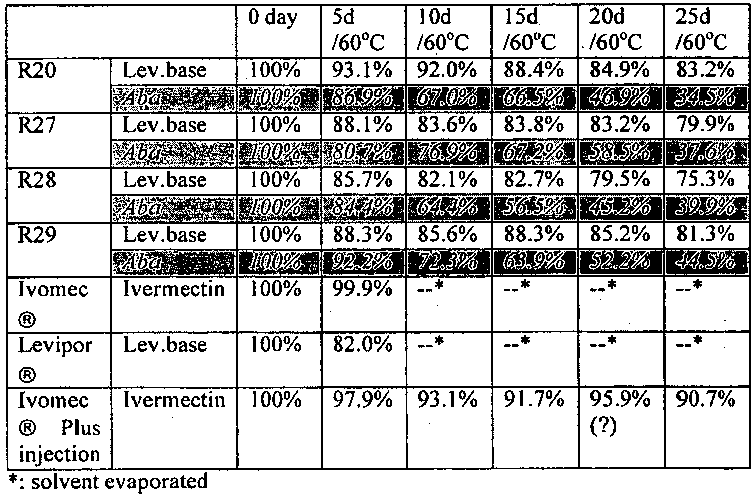

In all test formulations at elevated temperatures the abamectin component degraded significantly over the period of the study. The ivermectin component of the commercially available Ivomec® Plus formulation did not deteriorate to anywhere near the same extent as the abamectin component of the test formulations.

Whilst the levamisole component also deteriorated it did so at a much lower rate. The study once again demonstrated the difficulty of combining the two actives and that the presence of levamisole was very problematic in preparing the combination formulation.

### Study 3

A further range of formulations were prepared in which benzyl alcohol was used to solubilise the abamectin component of the formulations.

### Formulations

| Ingredients | Concentration (%, w/v) | | | | | |
|---|---|---|---|---|---|---|
| | 029/0 | 029/1 | 029/2BH T | 029/3/BH T | 029/4BH A | 029/5/BH A |
| Lev.base | 20.0 | 20.0 | 20.0 | 20.0 | 20.0 | 20.0 |
| Abamectin | 1.0 | 1.0 | 1.0 | 1.0 | 1.0 | 1.0 |
| Propylene Glycol | 41.0 | 41.0 | 41.0 | 41.0 | 41.0 | 41.0 |
| Benzyl Alcohol | -- | 15.0 | 15.0 | 15.0 | 15.0 | 15.0 |
| Isopropyl myristate | 4.0 | 4.0 | 4.0 | 4.0 | 4.0 | 4.0 |
| BHT | -- | -- | 0.2 | 1.0 | -- | -- |
| BHA | -- | -- | -- | -- | 0.2 | 1.0 |
| Diethylene glycol monoethyl ether to | 100ml | 100ml | 100ml | 100ml | 100ml | 100ml |

### Stability results

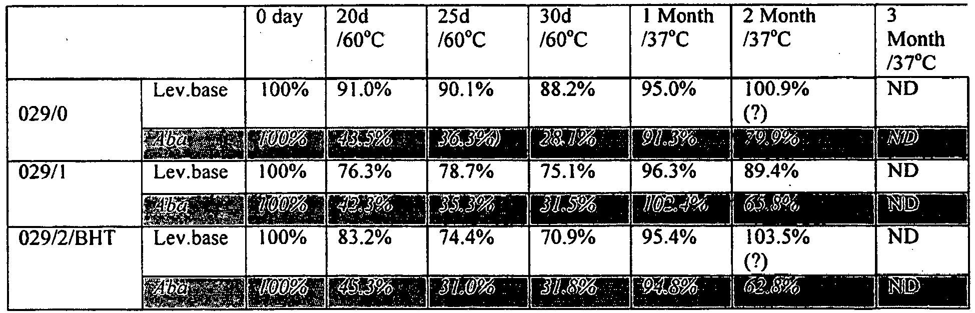

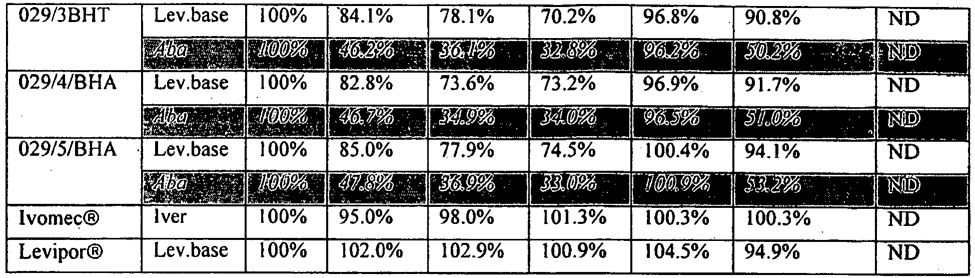

In the stability study the presence of benzyl alcohol did not have any significant effect in minimizing the rate of degradation of the abamectin component of the formulations. BHA and BHT also did not offer any advantage as stabilizing aids.

### Study 4

A study was undertaken to determine whether the use of propylene glycol or glycol ethers would have any advantage in stabilizing the formulations.

Two formulations were prepared these are shown in the table below.

### Formulations

| | R3 | R4 |
|---|---|---|
| Levamisole base | 20.0 g | 20.0 g |
| Abamectin | 1.0 g | 1.0 g |
| Propylene glycol | -- | 40 ml |
| *DGBE to | 100 ml | 100 ml |

| | | |
|---|---|---|
| *DGBE: Diethylene glycol n-butyl ether (Butyl carbitol^{®}) | | |

### Stability results

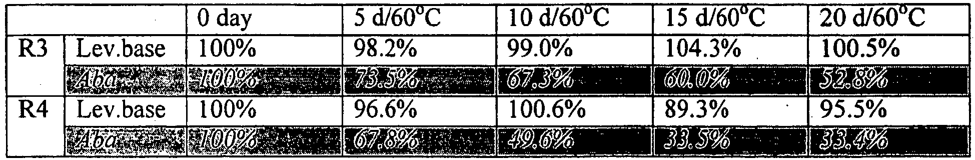

While levamisole base was relatively stable in both formulations the abamectin degraded in both formulations with the rate of degradation much more significant in the formulation that included propylene glycol. This suggested that propylene glycol was probably not beneficial in enhancing the stability of abamectin when used with DGBE.

### Study 5

A study was undertaken to attempt to improve the stability of formulations that used DGBE as their base.

### Formulations

| | 3-1 | 3-2 | 3-3 |
|---|---|---|---|
| Aba | 1.0 g | 1.0 g | 1.0 g |
| Leva.base | 20.0 g | 20.0 g | 20.0 g |
| BHT | - | 0.2 g | 2.0 g |
| *DGBE to | 100 ml | 100 ml | 100 ml |

| | | | |
|---|---|---|---|
| *DGBE: Diethylene Glycol n-butyl Ether | | | |

### Stability results

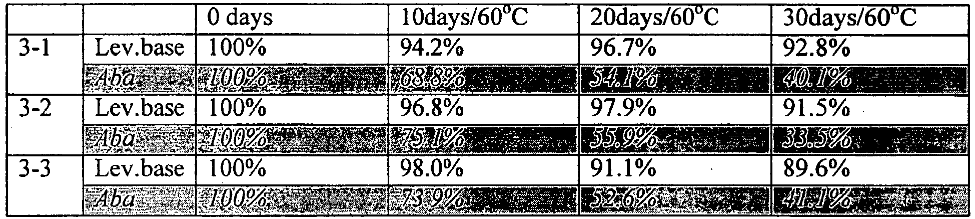

The study demonstrated that both BHT and BHA had no significant effect on enhancing the stability of the abamectin component of the formulation.

### Study 6

Alternate formulations that used benzoic acid and/or BHT were prepared to evaluate their effects on the stability of DGBE based formulations.

### Formulations

| | R1 | R2 | R3 | R4 | R5 | R6 |
|---|---|---|---|---|---|---|
| Lev. base | 20.0 g | 20.0 g | 20.0 g | 20.0 g | 20.0 g | 20.0 g |
| Abamectin | 1.0 g | 1.0 g | 1.0 g | 1.0 g | 1.0 g | 1.0 g |
| BHT | -- | -- | -- | 0.2 g | 0.2 g | 0.2 g |
| Benzoic acid | -- | 0.05 g | 0.2 g | -- | 0.05 g | 0.2 g |
| *DGBE to | 100 ml | 100 ml | 100 ml | 100 ml | 100 ml | 100 ml |

| | | | | | | |
|---|---|---|---|---|---|---|
| *DGBE: Diethylene Glycol n-butyl Ether | | | | | | |

### Stability results

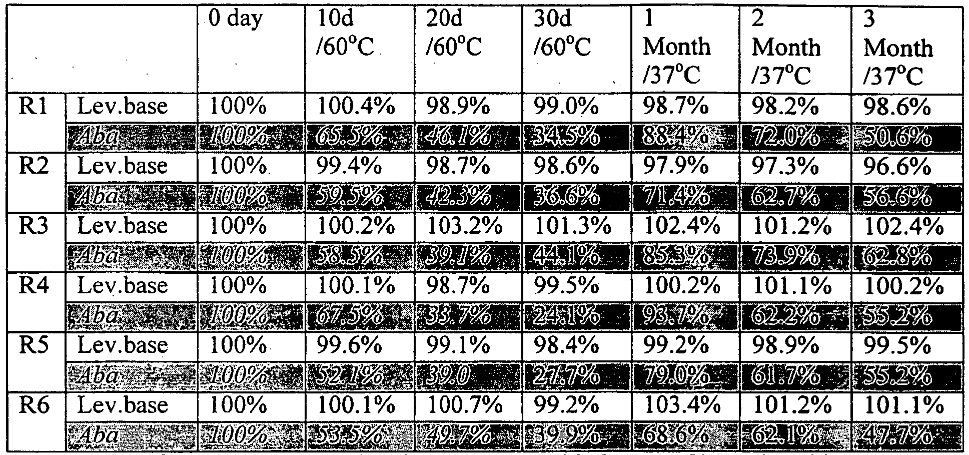

The stability of Abamectin showed no improvement with the use of benzoic acid or BHT.

### Study 7

A selection of new formulations that included other excipients with DGBE were prepared.

### Formulations

| | R3 | R4 | R5 | R6 |
|---|---|---|---|---|
| Lev.base | 20.0g | 15.0g | 20.0g | 20.0g |
| Lev.HCl | -- | 5.0g | -- | -- |
| Aba | 1.0g | 1.0g | 1.0g | 1.0g |
| β-CD | 0.5g | -- | -- | -- |
| Benzoic acid | -- | -- | 5.0g | -- |
| Citric acid | -- | -- | -- | 3.0g |
| Propylene Glycol | 40ml | 40ml | -- | -- |
| Glycerin Formal | 30ml | 30ml | -- | -- |
| Capmul MCM | -- | to 100ml | -- | -- |
| DGBE | to 100ml | -- | to 100ml | to 100ml |

| | | | | |
|---|---|---|---|---|
| DGBE: Diethylene glycol n-butyl ether | | | | |

| | R7 | R8 | R9 | R10 | R11-1 | R11-2 | R12 | R13 | R14 | R15 |
|---|---|---|---|---|---|---|---|---|---|---|
| Lev.base | 20.0 g | 20.0 g | 20.0 g | 20.0 g | 20.0 g | 20.0 g | 20.0 g | 20.0 g | 20.0 g | 20.0 g |
| Aba | 1.0g | 1.0g | 1.0g | 1.0g | 1.0g | 1.0g | 1.0g | 1.0g | 1.0g | 1.0g |
| TEA | -- | -- | -- | 1.0m l | -- | -- | 1.0m l | 1.0m l | -- | -- |
| EDTA | -- | -- | -- | -- | 0.01 g | -- | 0.01 g | 0.01 g | 0.01 g | 0.01 g |
| EDTA-2Na | -- | -- | -- | -- | -- | 0.01 g | -- | -- | -- | -- |
| BHT | -- | -- | -- | -- | 2.0g | 2.0g | 2.0g | -- | 2.0g | -- |
| BHA | -- | -- | -- | -- | -- | -- | -- | 2.0g | -- | 2.0g |
| Benzoic acid | -- | -- | -- | -- | -- | -- | -- | -- | 5.0g | 5.0g |
| DGMEE | to 100 ml | -- | -- | to 100 ml | to 100 ml | to 100 ml | To 100 ml | to 100 ml | to 100 ml | to 100 ml |
| DGBE | -- | to 100 ml | -- | -- | -- | -- | -- | -- | -- | -- |
| DPM | -- | -- | to 100 ml | -- | -- | -- | -- | -- | -- | -- |

| | | | | | | | | | | |
|---|---|---|---|---|---|---|---|---|---|---|
| TEA: Triethylamine; EDTA: Ethylenediaminetetraacetic acid; BHT: Butylated Hydroxy Tolueue; BHA: Butylated Hydroxyanisole; DGMEE: Diethylene glycol monoethyl ether; DGBE: Diethylene glycol n-butyl ether; DPM: Dipropylene glycol methyl ether | | | | | | | | | | |

### Stability results

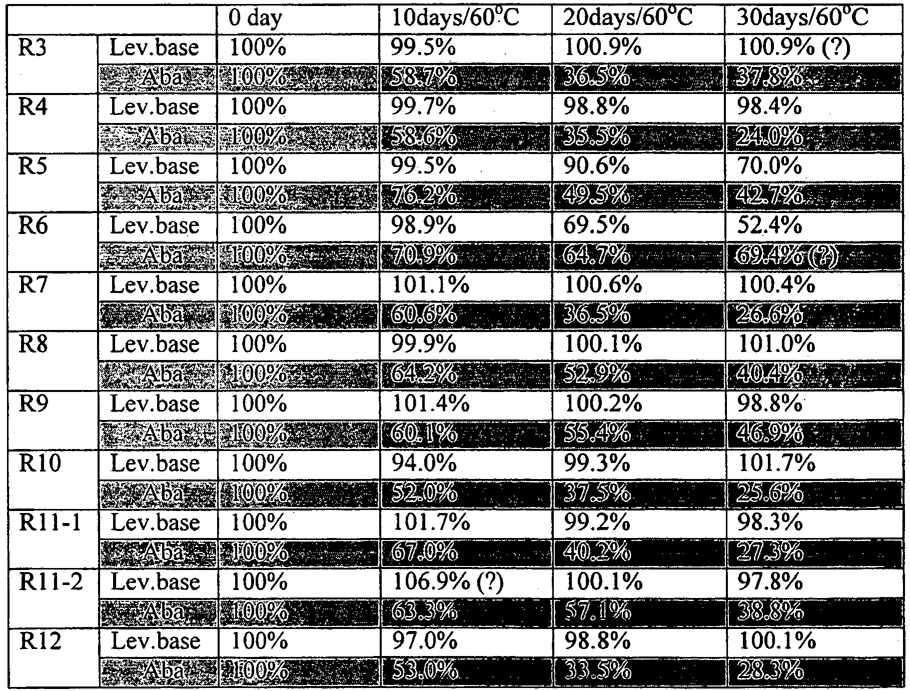

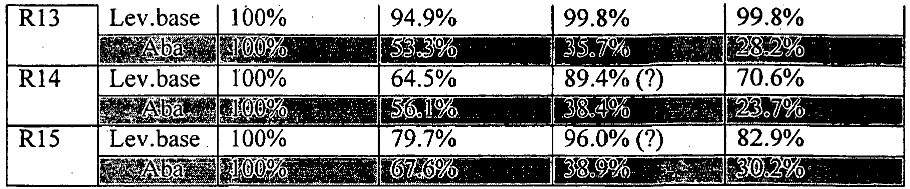

None of the formulations showed great promise in stabilizing the abamectin component of the formulations.

### Study 8

A selection of new formulations that included other excipients with DGMEE were prepared.

### Formulations

| | F1 | F2 | F3 | F4 | F5 | F6 | F7 | F8 |
|---|---|---|---|---|---|---|---|---|
| Lev.base | 20.0g | 20.0g | 20.0g | 20.0g | 20.0g | 20.0g | 20.0g | 20.0g |
| Abamectin | 1.0g | 1.0g | 1.0g | 1.0g | 1.0g | 1.0g | 1.0g | 1.0g |
| TEA | -- | 1.0ml | 1.0ml | -- | 1.0ml | 1.0ml | -- | -- |
| EDTA | -- | -- | -- | -- | -- | -- | 0.01g | 0.01g |
| H₂O | -- | -- | -- | 10g | 10g | 10g | -- | 10g |
| BHT | -- | -- | -- | -- | -- | -- | 2.0g | 2.0g |
| BHA | -- | -- | -- | -- | -- | -- | -- | -- |
| Benzoic Acid | 5.0g | - | 5.0g | 5.0g | -- | 5.0g | -- | -- |
| DGMEE to | 100ml | 100ml | 100ml | 100ml | 100ml | 100ml | 100ml | 100ml |

| | | | | | | | | |
|---|---|---|---|---|---|---|---|---|
| TEA: Triethylamine; EDTA: Ethylenediaminetetraacetic acid; BHT: Butylated Hydroxy Toluene; BHA: Butylated Hydroxyanisole; DGMEE: Diethylene glycol monoethyl ether | | | | | | | | |

### Stability results

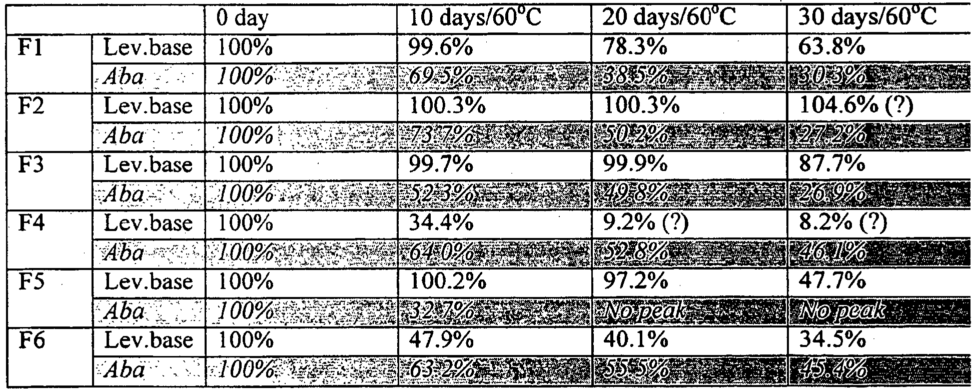

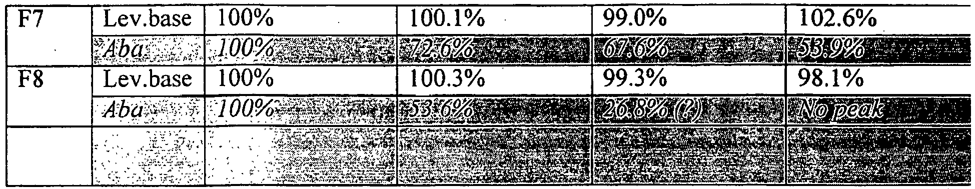

Once again none of the formulations showed great promise in stabilizing the abamectin component of the formulations.

### Study 9

Further alternate formulations were prepared according to table below.

### Formulations

| | R1 | R2 | R3 | R4 | R5 | R6 |
|---|---|---|---|---|---|---|
| Lev.base | 20.0g | 20.0g | 20.0g | 20.0g | 20.0g | 20.0g |
| Abamectin | 1.0g | 1.0g | 1.0g | 1.0g | 1.0g | 1.0g |
| Benzoic Acid | 5.0g | 5.0g | 5.0g | 10.0g | -- | -- |
| Acetic acid | -- | -- | -- | -- | 2.0ml | 4.0ml |
| BHA | -- | -- | 2.0g | -- | -- | -- |
| DGMEE to | 100ml | -- | -- | -- | -- | -- |
| DGBE to | -- | 100ml | 100ml | 100ml | 100ml | 100ml |

| | | | | | | |
|---|---|---|---|---|---|---|
| BHA: Butylated Hydroxyanisole; DGMEE: Diethylene glycol monoethyl ether; DGBE: Dithylene glycol n-butyl ether | | | | | | |

### Stability results

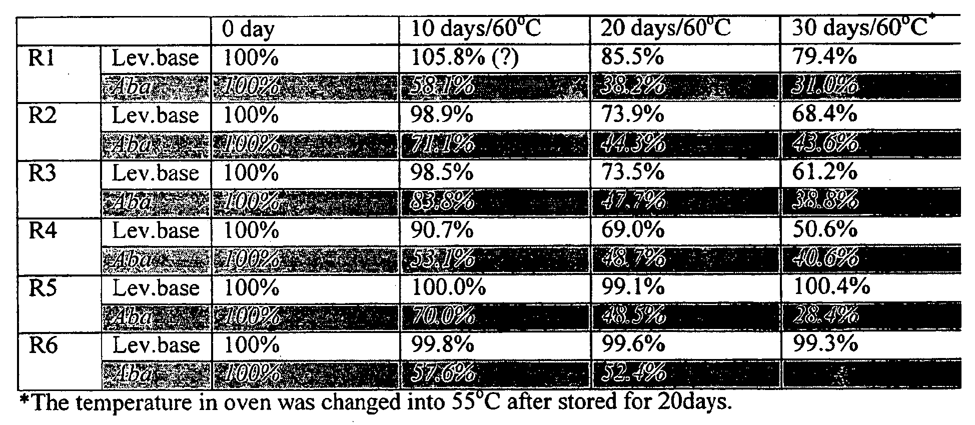

However none of these demonstrated great promise in stabilizing the abamectin component of the formulations.

### Study 10

Example formulations were prepared according to the table below.

### Formulations

| | R1 | R2 | R3 | R4 | R5 |
|---|---|---|---|---|---|
| Lev.base | 20.0 g | 20.0 g | 20.0 g | 20.0 g | 20.0 g |
| Abamectin | 1.0 g | 1.0 g | 1.0 g | 1.0 g | 1.0 g |
| Acetic acid | -- | 2.0ml | 4.0 ml | 6.0 ml | 10.0 ml |
| *DGBE to | 100 ml | 100 ml | 100 ml | 100 ml | 100 ml |

| | | | | | |
|---|---|---|---|---|---|
| *DGBE: Diethylene glycol n-butyl ether | | | | | |

### Stability results

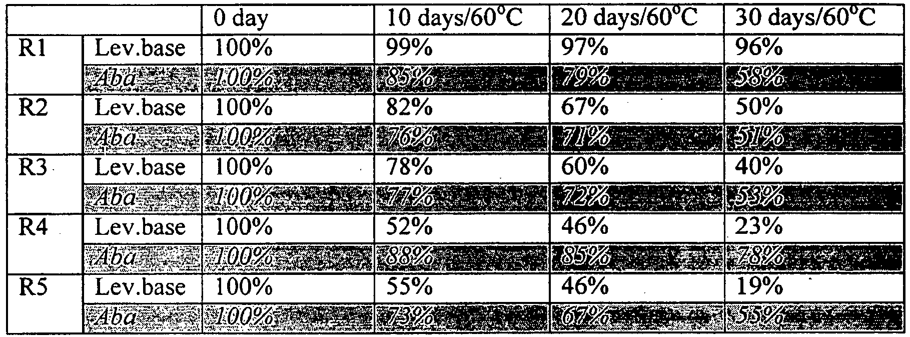

Formulations containing acetic acid did not improve the stability of abamectin. However, the stability of levamisole base was adversely affected to a significant extent.

### Study 11

A trial was carried out to determine whether the addition of varying levels of N-Methyl-2-Pyrollidone (Pharmasolv) to DGBE would enhance stability. All the formulations were kept at 60°C and were analysed to assess the extent of degradation after 7, 14 and 30 days.

### Formulations

| | G1 | G2 | G3 | G4 | G5 |
|---|---|---|---|---|---|
| Lev.base | 20.0% w.v. | 20.0% w/v | 20.0% w/v | 20.0% w/v | 20.0% w/v |
| Abamectin | 1.15% w/v | 1.15% w/v | 1.15% w/v | 1.15% w/v | 1.15% w/v |
| DGBE | - | 25% w/v | 40% w/v | q/v. | q.v. |
| N-Methyl-2-Pyrollidone | q.v. | q.v | q.v | 25% | - |

### Stability Results

| Form. | Initial | | 7 days at 60°C | | 14 days at 60°C | | 1 month at 60° | |
|---|---|---|---|---|---|---|---|---|
| | Abamecti n | Levamisole | Abamectin | Levamisole | Abamectin | Levamisole | Abamectin | Levamisole |
| G1 | 96.12 | 101.43 | 93.04 | 95.55 | 89.57 | 89.75 | 79.13 | 86.95 |
| G2 | 100.24 | 103.22 | 95.65 | 99.50 | 95.65 | 96.35 | 79.13 | 93.60 |
| G3 | 103.30 | 102.58 | 93.91 | 97.00 | 87.83 | 95.20 | 66.96 | 92.85 |
| G4 | 109.05 | 101.70 | 101.74 | 99.95 | 93.91 | 99.35 | 66.57 | 93.80 |
| G5 | 89.42 | 100.32 | 83.48 | 97.80 | 80.00 | 93.30 | 57.39 | 89.55 |

The stability results of the solution containing both the actives in Pharmasolv demonstrated that surprisingly a pyrollidone based formulation was capable of significantly slowing the rate of degradation of both levamisole and abamectin.

To further confirm the findings of this study new batches were prepared with the formulation as specified in the following table:

| *Material* | *Formulation* |
|---|---|
| Lev.base | 20.0% w/v |
| Abamectin | 1.15% w/v |
| DGBE | 25% w/v |
| N-Methyl-2-Pyrollidone | q.v |

Stability results over a twelve month period of storage at 25°C confirmed the increased stability of an abamectin/levamisole formulation containing N-Methyl-2-Pyrollidone (Pharmasolv) and DGBE.

| *ACTIVE* | *Initial* | *6 Month* | *12 Months* |
|---|---|---|---|
| Abamectin | 104.00 | 102.55 | 99.95 |
| Levamisole | 99.75 | 99.00 | 98.55 |

### Field Studies

The formulation of the table above containing DGBE and N⁻methyl-2- pyrollidone was used in a slaughter study to evaluate the effectiveness of the formulation relative to formulations containing either levamisole or an avermectin or milbemycin The results clearly demonstrated that whilst the levamisole-based formulation (Levipor®) performed poorly against *Ostertagia* and the eprinomectin-based formulation (Eprinex®) performed poorly against *Cooperia,* the abamectin/levamisole combination showed outstanding efficacy against all parasite species

A large number of field studies on cattle of all ages have also confirmed that m contrast with a number of the other test formulations there is no skin irritation on treated animals.

**Table 1: Geometric mean total worm counts for calves treated with Abamectin / levamisole pour-on, Eprinex® pour-on or Levipor® pour-on in comparision with an untreated control group**

| **Treatment** | **Control** | **Aba/Lev PO** | **Eprinex® PO** | **Levipor® PO** |
|---|---|---|---|---|
| *Ostertagia* (adult) | 11435.5^{a} | 4.4^{b} | 17.3^{b} | 5808.1^{a} |
| *Ostertagia* (immature) | 1274^{a} | 2.3^{b} | 0^{b} | 13174^{a} |
| *T. axei* (*adult)* | 996.7^{a} | 0^{b} | 0^{b} | 110.9^{a} |
| *T.axei* (immature) | 4.7^{a} | 0^{a} | 0^{a} | 1.9^{a} |
| *Trichostrongylus* spp (mature) | 744.3^{a} | 6.7^{b} | 46.4^{a} | 5^{b} |
| *Cooperia* (adult) | 15948 8^{a} | 1.9^{b} | 2155.8^{a} | 5.9^{b} |
| *Cooperia* (immature) | 1598.7^{a} | 1.9^{b} | 5.7^{b} | 1.9^{b} |
| *Oesophagostomum* (mature) | 2.5^{a} | 0^{a} | 0^{a} | 0^{a} |
| *Trichuris* (mature) | 35.4^{a} | 0^{b} | 0^{b} | 0^{b} |

| | | | | |
|---|---|---|---|---|
| ^{a} means within the same row with different superscripts are significantly different at p<0 05 | | | | |

**Table 2: Treatment efficacies based on group geometric mean total worm counts**

| **Treatment** | **Aba/Lev PO** | **Eprinex® PO** | **Levipor® PO** |
|---|---|---|---|
| *Ostertagia* (adult) | >99.9% | 99.8% | 49.2% |
| *Ostertagia* (immature) | 99.8% | >99.9% | 0% |
| *T. axei* (adult) | >99.9% | >99.9% | 80.1% |
| *T.axei* (immature) | >99.9% | >99.9% | >99 9% |
| *Trichostrongylus* spp (mature) | 99.1% | 93.7% | 99.3% |
| *Cooperia* (adult) | >99.9% | 865% | >99 9% |
| *Cooperia* (immature) | 99.8% | 99.6% | 99.9% |
| *Oesophagostomum* (mature) | >99.9% | >99.9% | >99.9% |
| *Trichuris* (mature) | >99.9% | >99.9% | >99.9% |

### PREFERRED EMBODIMENTS

In the preferred embodiments the formulations of the invention there include avermectin or milbemycin in combination with levamisole and a pyrrolidone solvent. A glycol ether may additionally be included.

The following examples are provided as examples only and are in no way intended to limit the spirit or scope of the invention.

### Example Formulations

The formulations of the present invention are prepared as follows:
1. Add levamisole base, avermectin/milbemycin and pyrollidone to a mixing vessel.
2. Stir at room temperature until the actives have completely dissolved.
3. Add the glycol ether, if desired, and mix well.
4. Add the pyrolidone to volume and continue mixing until a clear solution is obtained.

### Topical Formulations

### 1. Examples of topically applied formulations of the invention include:

### Formulation 1.1

| *Ingredient* | *% w*/*v* |
|---|---|
| Abamectin | 1% |
| Levamisole Base | 20% |
| n-methyl pyrrolidone | q.v. |

### Formulation 1.2

| *Ingredient* | *% w*/*v* |
|---|---|
| Ivermectin | 0.5% |
| Levamisole Base | 10% |
| n-methyl pyrrolidone | q.v. |

### Formulation 1.3

| *Ingredient* | *% w*/*v* |
|---|---|
| Ivermectin | 0.5% |
| Levamisole Base | 10% |
| DGMBE | 25% |
| n-methyl pyrrolidone | q.v. |

### Formulation 1.4

| *Ingredient* | *% w*/*v* |
|---|---|
| Eprinomectin | 1.0% |
| Levamisole Base | 20% |
| DGMBE | 25% |
| n-methyl pyrrolidone | q.v. |

### 2. Examples of Injectable formulations include:

### Formulation 2.1

| *Ingredient* | *% w*/*v* |
|---|---|
| Ivermectin | 0.5% |
| Levamisole Phosphate | 20% |
| 2- pyrrolidone | q.v. |

### Formulation 2.2

| *Ingredient* | *% w*/*v* |
|---|---|
| Moxidectin | 0.5% |
| Levamisole Phosphate | 20% |
| 2 - pyrrolidone | q.v. |

### 3. Examples of Orally administered formulations include:

### Formulation 3.1

| *Ingredient* | *% w*/*v* |
|---|---|
| Abamectin | 0.1% |
| Levamisole Base | 5% |
| n-methyl pyrrolidone | q.v. |

### Formulation 3.2

| *Ingredient* | *% w*/*v* |
|---|---|
| Ivermectin | 1% |
| Levamisole Base | 5% |
| n-methyl pyrrolidone | q.v. |

### Formulation 3.3

| *Ingredient* | *% w*/*v* |
|---|---|
| Abamectin | 1% |
| Levamisole Base | 20% |
| n-methyl pyrrolidone | q.v. |

### Formulation 3.4

| *Ingredient* | *% w*/*v* |
|---|---|
| Abamectin | 1% |
| Levamisole Base | 20% |
| n-methyl pyrrolidone | q.v. |

### Formulation 3.5

| *Ingredient* | *% w*/*v* |
|---|---|
| Abamectin | 1% |
| Levamisole Base | 20% |
| n-methyl pyrrolidone | q.v. |

The rates for these formulations are generally in the order of 1ml to 5kg to 1ml per 20kg for oral administration, 1ml per 25 kg or 1ml per 50kg for administration by injection, and 1ml per 10kg or 1 ml per 20kg for topical administration.

The methods of administration of the formulations are well known within the art.

## Claims

1. A stable formulation suitable for administration to animals including at least one first active selected from avermectins and milbemycins, and a second active which is levamisole, said actives being dissolved in a pyrrolidone solvent.

2. A stable formulation suitable for administration to animals as claimed in claim 1, additionally including a solvent selected from the glycol ethers.

3. A stable formulation suitable for administration to animals as claimed in any previous claim, wherein the pyrrolidone solvent is 2-pyrolidone or N-methyl pyrrolidone.

4. A stable formulation suitable for administration to animals as claimed in any previous claim, wherein the avermectin or milbemycin is present in the range of between 0.01 - 5% w/v.

5. A stable formulation suitable for administration to animals as claimed in claim 4, wherein the avermection or milbemycin is selected from abamectin, doramectin, eprinomectin, ivermectin and moxidectin.

6. A stable formulation suitable for administration to animals as claimed in any previous claim, wherein the levamisole is present in the range of between 1 - 30% w/v.

7. A stable formulation suitable administration to animals as claimed in any previous claim, wherein the formulation additionally includes at least one further medicament selected from anthelmintics, dietary supplements, vitamins, mineral and other beneficial agents.

8. A stable formulations suitable for administration to animals as claimed in any previous claim, wherein the formulation is suitable for topical administration.

9. A stable formulation suitable for administration to animals as claimed in any of claims 1 to 8, wherein the formulation is suitable for parenteral administration.

10. A stable formulation suitable for administration to animals as claimed in any of claims 1 to 8, wherein the formulation is suitable for oral administration.

11. Use of a formulation as claimed in any of claims 1-10 in the preparation of a medicament for treating infection of cattle with *Cooperia* or *Ostertagia.*

12. A formulation as claimed in any of claims 1-10 for use in treating infection of cattle with *Cooperia* or *Ostertagia.*

## Patentansprüche

1. Eine stabile Formulierung geeignet zur Verabreichung an Tiere, enthaltend mindestens einen ersten Wirkstoff ausgewählt aus Avermectinen und Milbemycinen und einen zweiten Wirkstoff, der Levamisol ist, wobei die Wirkstoffe in einem Pyrrolidon-Lösungsmittel gelöst sind.

2. Eine stabile Formulierung geeignet zur Verabreichung an Tiere, wie in Anspruch 1 beansprucht, die zusätzlich ein Lösungsmittel ausgewählt aus Glycolethern enthält.

3. Eine stabile Formulierung geeignet zur Verabreichung an Tiere, wie in einem vorhergehenden Anspruch beansprucht, wobei es sich bei dem Pyrrolidon-Lösungsmittel um 2-Pyrrolidon oder N-Methylpyrrolidon handelt.

4. Eine stabile Formulierung geeignet zur Verabreichung an Tiere, wie in einem vorhergehenden Anspruch beansprucht, wobei das Avermectin oder Milbemycin im Bereich zwischen 0,01 - 5 % Gewicht/Volumen vorliegt.

5. Eine stabile Formulierung geeignet zur Verabreichung an Tiere, wie in Anspruch 4 beansprucht, wobei das Avermectin oder Milbemycin aus Abamectin, Doramectin, Eprinomectin, Ivermectin und Moxidectin ausgewählt ist.

6. Eine stabile Formulierung geeignet zur Verabreichung an Tiere, wie in einem vorhergehenden Anspruch beansprucht, wobei das Levamisol im Bereich zwischen 1 - 30 % Gewicht/Volumen vorliegt.

7. Eine stabile Formulierung geeignet zur Verabreichung an Tiere, wie in einem vorhergehenden Anspruch beansprucht, wobei die Formulierung zusätzlich mindestens ein weiteres Medikament, ausgewählt aus Anthelmintika, Nahrungsergänzungsmitteln, Vitaminen, Mineralien und anderen Wirkstoffen, enthält.

8. Eine stabile Formulierung geeignet zur Verabreichung an Tiere, wie in einem vorhergehenden Anspruch beansprucht, wobei die Formulierung zur topischen Verabreichung geeignet ist.

9. Eine stabile Formulierung geeignet zur Verabreichung an Tiere, wie in einem der Ansprüche 1 bis 7 beansprucht, wobei die Formulierung zur parenteralen Verabreichung geeignet ist.

10. Eine stabile Formulierung geeignet zur Verabreichung an Tiere, wie in einem der Ansprüche 1 bis 7 beansprucht, wobei die Formulierung zur oralen Verabreichung geeignet ist.

11. Verwendung einer Formulierung wie in einem der Ansprüche 1 - 10 beansprucht zur Herstellung eines Medikaments zur Behandlung von Infektionen bei Rindern mit *Cooperia* oder *Ostertagia.*

12. Eine Formulierung wie in einem der Ansprüche 1 - 10 beansprucht zur Verwendung bei der Behandlung von Infektionen bei Rindern mit *Cooperia* oder *Ostertagia.*

## Revendications

1. Formulation stable appropriée pour l'administration à des animaux comprenant au moins un premier agent actif choisi parmi les avermectines et les milbémycines, et un deuxième agent actif qui est le lévamisole, lesdits agents actifs étant dissous dans un solvant pyrrolidone.

2. Formulation stable appropriée pour l'administration à des animaux selon la revendication 1, comprenant en outre un solvant choisi parmi les éthers de glycol.

3. Formulation stable appropriée pour l'administration à des animaux selon l'une quelconque des revendications précédentes, dans laquelle le solvant pyrrolidone est la 2-pyrrolidone ou la N-méthyl-pyrrolidone.

4. Formulation stable appropriée pour l'administration à des animaux selon l'une quelconque des revendications précédentes, dans laquelle l'avermectine ou la milbémycine est présente en un ordre de grandeur entre 0,01 et 5 % p/v.

5. Formulation stable appropriée pour l'administration à des animaux selon la revendication 4, dans laquelle l'avermectine ou la milbémycine est choisie parmi l'abamectine, la doramectine, l'éprinomectine, l'ivermectine et la moxidectine.

6. Formulation stable appropriée pour l'administration à des animaux selon l'une quelconque des revendications précédentes, dans laquelle le lévamisole est présent en un ordre de grandeur entre 1 et 30 % p/v.

7. Formulation stable appropriée pour l'administration à des animaux selon l'une quelconque des revendications précédentes, la formulation comprenant en outre au moins un autre médicament choisi parmi les anti-helminthiques, les compléments alimentaires, les vitamines, les minéraux et autres agents bénéfiques.

8. Formulation stable appropriée pour l'administration à des animaux selon l'une quelconque des revendications précédentes, la formulation étant appropriée pour l'administration topique.

9. Formulation stable appropriée pour l'administration à des animaux selon l'une quelconque des revendications précédentes 1 à 7, la formulation étant appropriée pour l'administration parentérale.

10. Formulation stable appropriée pour l'administration à des animaux selon l'une quelconque des revendications précédentes 1 à 7, la formulation étant appropriée pour l'administration orale.

11. Utilisation d'une formulation selon l'une quelconque des revendications 1 à 10, dans la préparation d'un médicament pour traiter une infection du bétail par *Cooperia* ou *Ostertagia.*

12. Formulation selon l'une quelconque des revendications 1 à 10, pour son utilisation dans le traitement d'une infection du bétail par *Cooperia* ou *Ostertagia.*
